# FASCICULE DE BREVET EUROPEEN

(11) **EP 0 295 350 B1**
(45) Date de publication et mention de la délivrance du brevet: **11.11.1992**
(21) Numéro de dépôt: 87401364.2
(22) Date de dépôt: 17.06.1987
(51) Int. Cl.: A61K 31/415

(54) **Application du N-[2-(diethylamino)ethyl]2-methoxy 4-[(1-H 4,5-dihydro-2-imidazolyl) amino]5-chloro benzamide comme anxiolytique et anti-psychotique**
Anwendung von N-[2-(diäthylamino)äthyl]-2-methoxy-4[(1-H-4,5-dihydro-2-imidazolyl)amino]-5-chlorobenzamid als anxiolytisches und antipsychotisches Mittel
Use of N-[2-(diethylamino)ethyl]-2-methoxy-4-[(1-H 4,5-dihydro-2-imidazolyl)amino]-5-chlorobenzamide as an anxiolytic and antipsychotic agent

(43) Date de publication de la demande: 21.12.1988
(73) Titulaire: Laboratoires DELAGRANGE, 91380 Chilly-Mazarin (FR)
(72) Inventeur: Acher, Jacques, F-91760 Itteville (FR); Monier, Jean-Claude, F-91510 Lardy (FR); Schmitt, Jean-Paul, F-91290 Arpajon (FR); Costall, Brenda, Ilkey West Yorshire (GB); Naylor, Robert, Ilkey West Yorshire (GB)
(74) Mandataire: Thouret-Lemaitre, Elisabeth

(56) Documents cités:
- EP-A- 0 236 646

## Description

La présente invention concerne l'utilisation du N-[2-(diéthylamino)-éthyl] 2-méthoxy 4-[1-H 4,5 dihydro 2-imidazolyl)amino] 5-chloro benzamide, de formule : 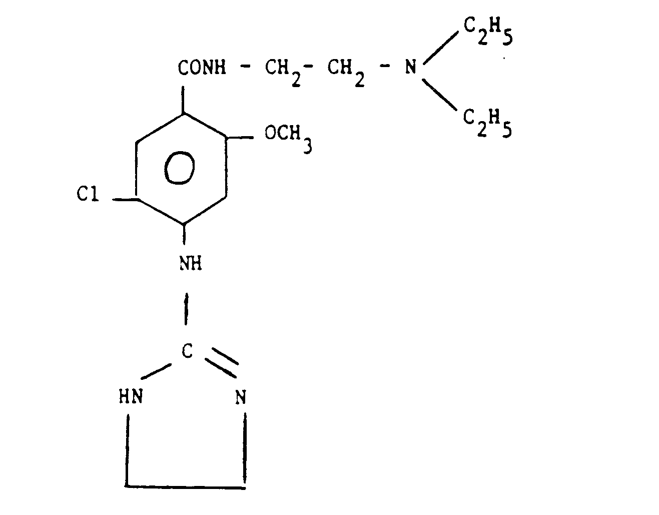
et de ses sels pharmacologiquement acceptables, pour la préparation d'un médicament utile comme anxiolytique et antipsychotique.

Ce composé est déjà connu comme activant du système nerveux central et antidépresseur, selon le brevet français n° FR-A-2 592 042. L'activité anti-dépressive se trouve d'ailleurs confirmée par le test de Porsolt.

Ce composé se distingue, en tant que psychotrope, d'autres composés methoxy-benzamides par le fait qu'il ne possède pas de propriétés antidopaminergiques. De ce fait, le composé selon l'invention n'est pas un neuroleptique au sens habituel du terme car il ne se lie pas aux récepteurs dopaminergiques D₁-D₂. Il est inactif dans les essais de comportement habituellement réalisés pour tester les neuroleptiques. Ces résultats ne pouvaient laisser supposer a priori que le composé selon l'invention soit tranquillisant et plus spécifiquement anxiolytique.

Une étude très approfondie du composé selon l'invention a montré qu'il possédait des propriétés inattendues :

### Anxiolytique

Le composé selon l'invention se révèle puissamment anxiolytique. Ceci est démontré par le test de la boîte à deux compartiments (sombre et éclairé) chez la souris, par l'observation du comportement "social" du rat, par le test de confrontation humaine chez le ouistiti. De plus, ce composé lève l'anxiété de sevrage de substances susceptibles de provoquer une pharmacodépendance telles que l'alcool, la nicotine, la cocaïne... A l'usage, ces substances procurent un effet anxiolytique. Cependant, leur utilisation chronique s'accompagne d'un état de dépendance. L'arrêt brusque de la prise de ces produits entraîne une exacerbation de l'anxiété initiale, rendant le sevrage de ces substances extrêmement difficile.

Les médicaments du type benzodiazépine, habituellement utilisés dans le traitement de l'anxiété présentent l'inconvénient d'induire à l'arrêt du traitement une exacerbation de l'anxiété et ne constituent donc pas une thérapie bien adaptée dans le sevrage des toxicomanes. Contrairement à cela, aucun effet anxiogène ne se manifeste lors de l'arrêt d'un traitement chronique par le composé selon l'invention.

### Anti-psychotique (schizophrénie)

A ce jour, les médicaments anti-psychotiques sont des antidopaminergiques, la théorie actuelle de la physio-pathologie de la schizophrénie étant une activité dopaminergique accrue au niveau du mésolimbe.
Les tests mis en application démontrent que le composé selon l'invention supprime l'hyperactivité induite par la dopamine perfusée dans le cerveau du rat, plus précisément dans le noyau accumbens.

Le protocole mis en oeuvre consiste en les tests suivants :

### 1. Test de la boîte à 2 compartiments chez la souris

Ce test est basé sur l'aversion naturelle des souris à l'égard de la lumière.

L'appareil utilisé est une boîte comprenant deux compartiments dont l'un est sombre (indiqué "compartiment noir" dans les figures) et l'autre éclairé (indiqué "compartiment blanc" dans les figures). Cet appareil est conçu pour laisser à l'animal le choix de séjourner dans l'un ou l'autre des compartiments. Dans des conditions normales, les souris évitent la lumière et restent surtout dans la partie sombre. Sous l'influence d'un anxiolytique, l'exploration du compartiment éclairé prédomine.

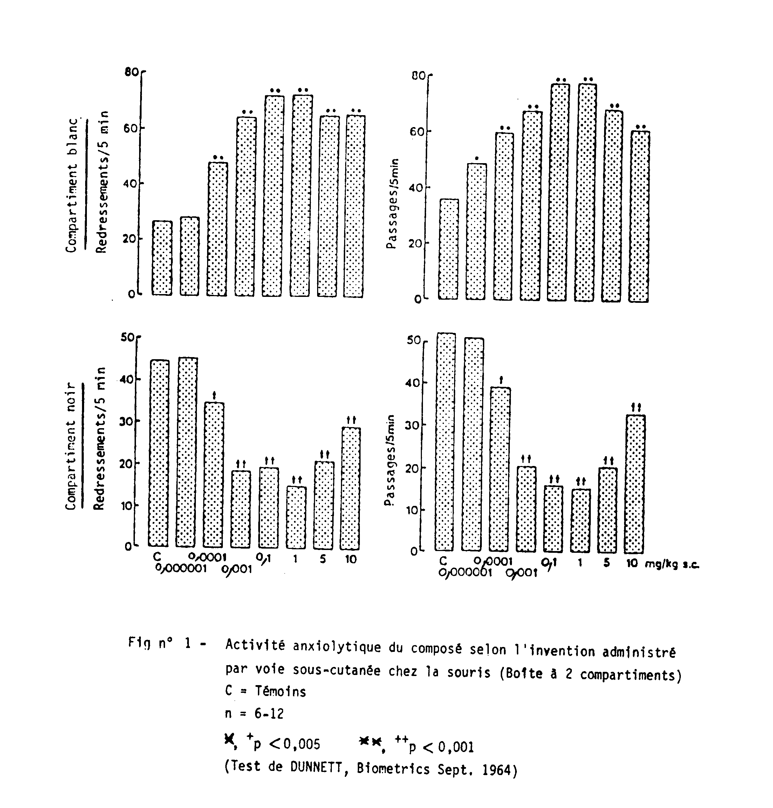

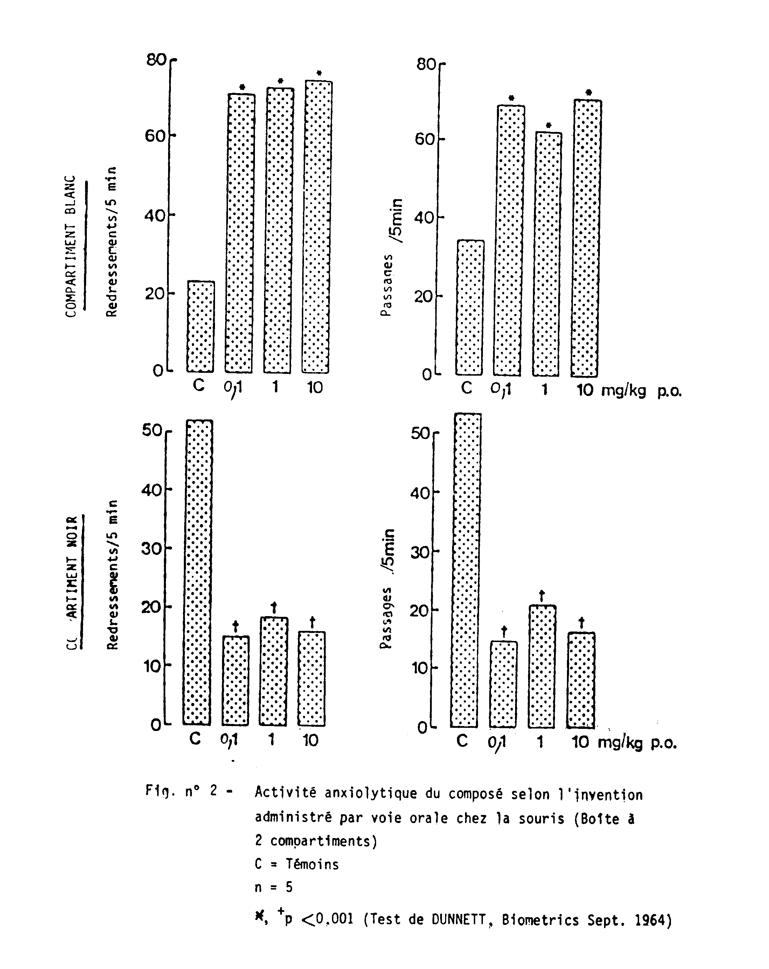

Le test consiste à déposer chaque souris au centre du compartiment éclairé et à observer le comportement de l'animal par système vidéo pendant 5 minutes. Le nombre de redressements exploratoires et le nombre de déplacements (franchissement de lignes tracées sur le plancher de chaque compartiment = passages) sont ensuite notés pour chaque compartiment.
- Un effet anxiolytique induit par un médicament tel que le diazepam se caractérise par une augmentation du nombre de redressements exploratoires et du nombre de passages dans le compartiment éclairé.
- Le produit selon l'invention exerce sur ce test un effet anxiolytique qui se manifeste à partir de 0,001 mg/kg par voie sous-cutanée et 0,1 mg/kg par voie orale par l'augmentation significative du nombre des redressements exploratoires et des déplacements dans le compartiment éclairé (Fig. n° 1 et no° 2).

### 2. Test de comportement "social" chez le rat

Ce test consiste à observer par système vidéo le comportement de 2 rats, issus de cages différentes, mis en présence l'un de l'autre, pendant 10 minutes, dans une boite fortement éclairée.

Le comportement "social" de chaque animal est évalué par la mesure de la durée de différentes activités : flair du partenaire, saut par dessus le partenaire, toilette, exploration génitale et filature du partenaire. Sous l'influence anxiogène de la lumière, ce comportement tend à se maintenir à un niveau réduit.

L'administration des substances anxiolytiques telles les benzodiazépines, permet la levée de cet effet, avec comme conséquence une augmentation du comportement social.

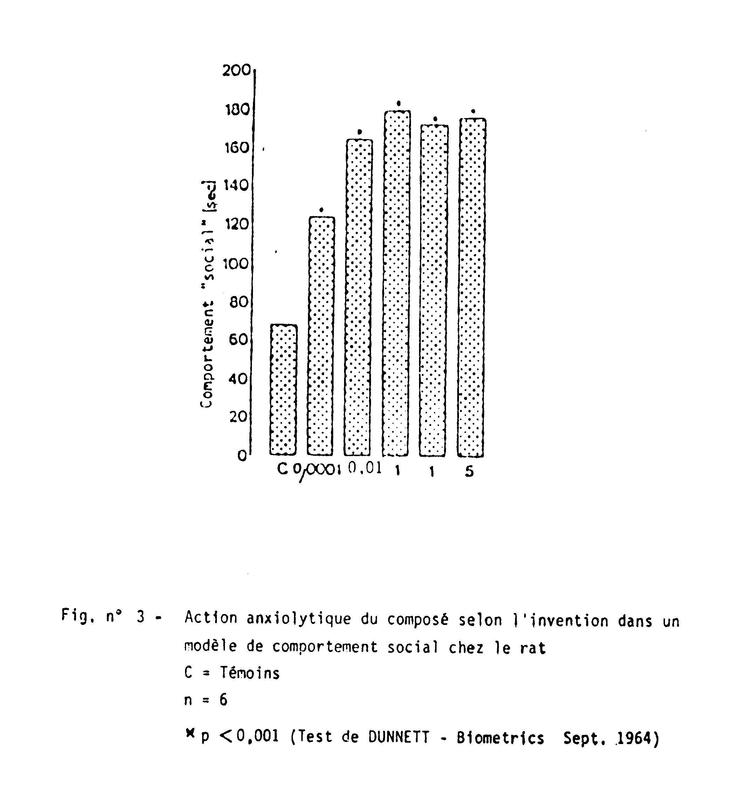

Le composé selon l'invention augmente le comportement social de façon statistiquement significative après l'administration d'une dose minimale de 0,0001 mg/kg par voie sous-cutanée (Fig. n° 3).

### 3. Test d'anxiété chez le ouistiti

Chez le ouistiti, la présence d'un expérimentateur inconnu se tenant debout à 60 cm de la cage de l'animal déclenche une anxiété qui se manifeste par une série de postures agressives et le repli de l'animal dans le fond de la cage.

L'administration d'un anxiolytique comme par exemple le diazepam réduit l'anxiété de l'animal et l'on observe une diminution du nombre de postures agressives ainsi qu'une augmentation du temps passé sur le devant de la cage, face à l'expérimentateur.

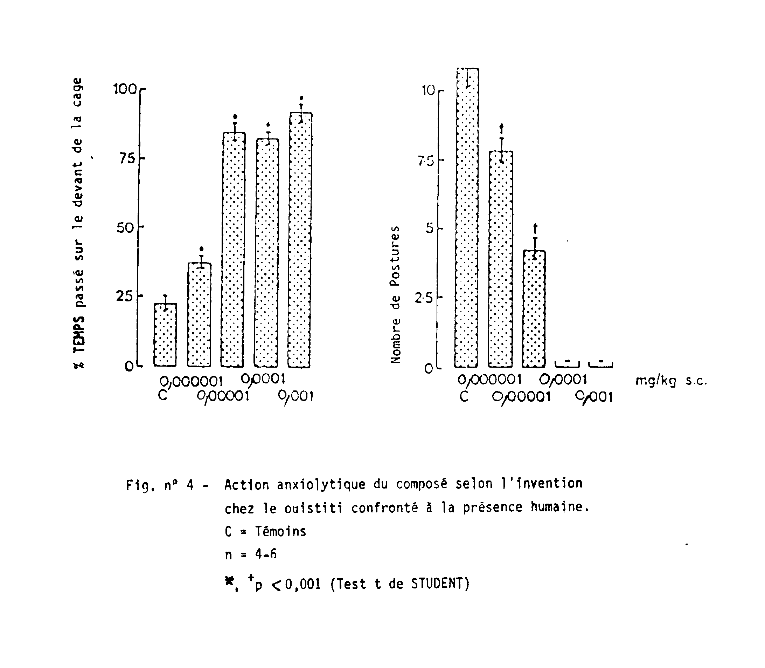

De la même façon, le composé selon l'invention réduit l'anxiété. Cet effet se manifeste par voie s.c. à partir de 0,000001 mg/kg par une diminution significative du nombre de postures agressives et une augmentation significative du temps passé sur le devant de la cage (Fig. n° 4).

### 4. Anxiété provoquée par le sevrage

Des souris rendues dépendantes par l'administration répétée de diverses substances subissent un sevrage (arrêt du traitement). L'anxiété provoquée par ce sevrage est objectivée comme précédemment décrit par le test de la boite à deux compartiments.

De façon générale, l'administration des substances, provoquant la dépendance, diminue "l'état anxieux" de l'animal. Cela se traduit par une augmentation du nombre de redressements et passages dans le compartiment éclairé. Lors du sevrage, l'on constate une exacerbation de l'anxiété de l'animal avec comme conséquence une augmentation du nombre de redressements et de passages dans le compartiment sombre qui dépassent les valeurs observées avant l'administration de la substance.

Le test consiste à administrer le produit à tester lors du sevrage jusqu'au jour de l'épreuve de la boîte à 2 compartiments, l'augmentation du nombre de passages et de redressements dans le compartiment éclairé étant le critère de l'efficacité du produit sur l'anxiété provoquée par le sevrage.

### Dépendance à l'alcool

Des souris rendues dépendantes à l'alcool par adjonction de 8% d'alcool à l'eau de boisson pendant 14 jours subissent un sevrage et sont testées 48 h après le début du sevrage (effet anxiogène maximal).

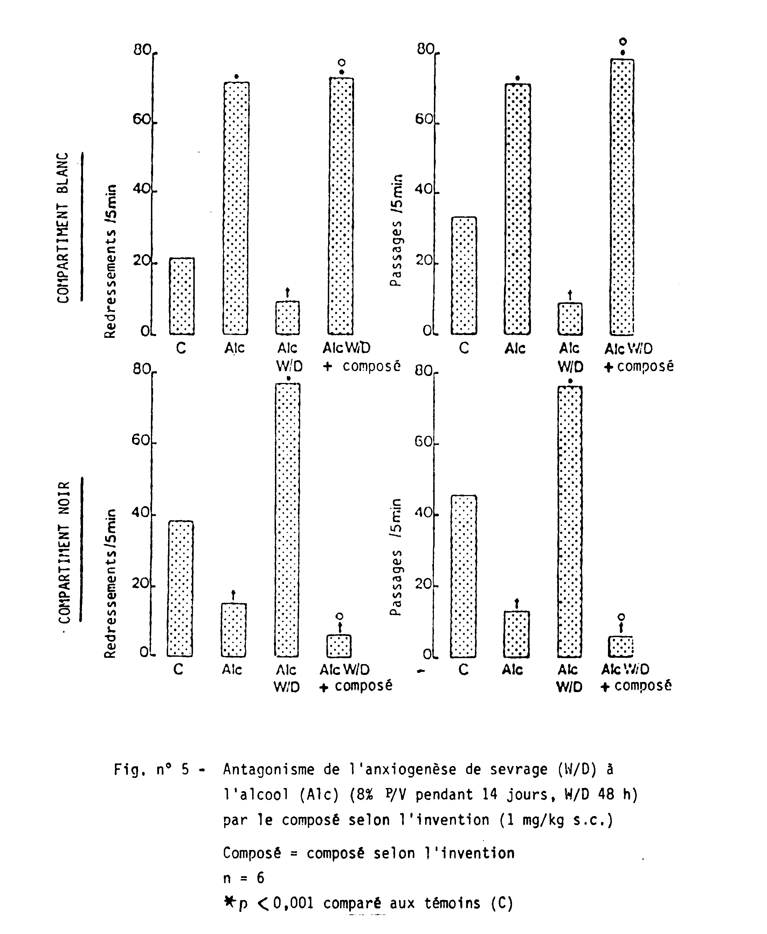

L'administration de 1 mg/kg/j du composé selon l'invention pendant les 48 heures suivant le sevrage, augmente de façon significative le nombre de passages et de redressements dans le compartiment éclairé par rapport aux animaux sevrés non traités (Fig. n° 5).

### Dépendance à la nicotine

Des souris rendues dépendantes à la nicotine par administration i.p. de 0,1 mg/kg x 2/j de nicotine pendant 14 jours subissent un sevrage et sont testées 48 h après le début du sevrage (effet anxiogène maximal).

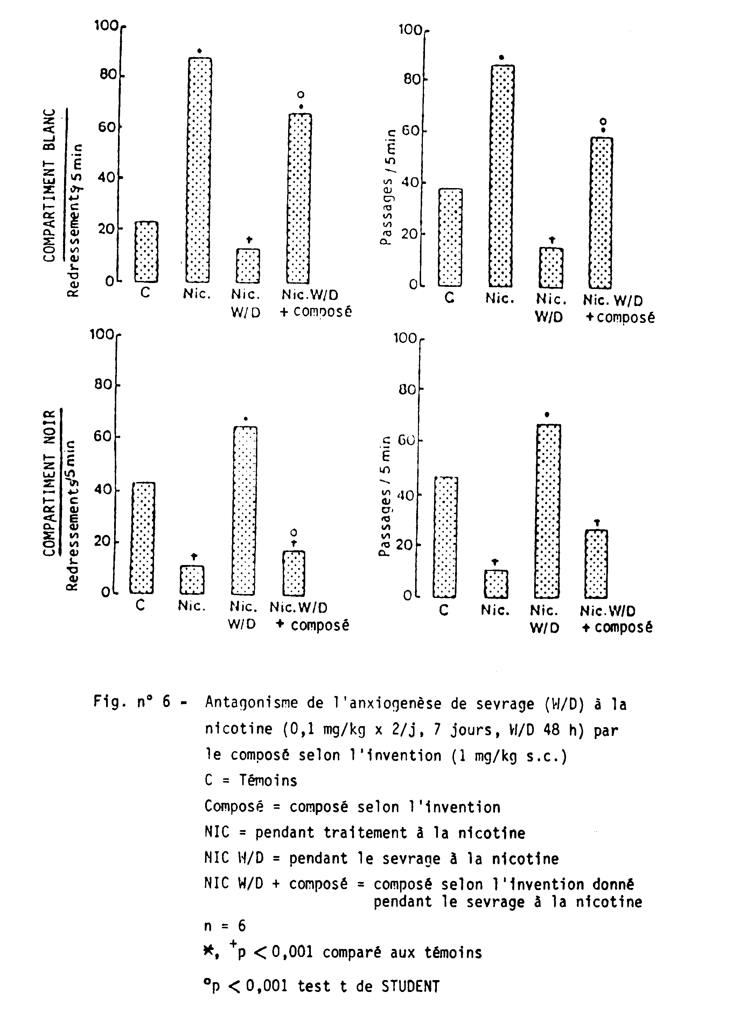

L'administration de 1 mg/kg/j du composé selon l'invention pendant les 48 heures suivant le sevrage, augmente de façon significative le nombre de passages et de redressements dans le compartiment éclairé par rapport aux animaux sevrés non traités (Fig. n° 6).

### Dépendance à la cocaïne

Des souris rendues dépendantes à la cocaïne par administration de 1 mg/kg x 2/j pendant 14 jours subissent un sevrage et sont testées 8 h après le début du sevrage (effet anxiogène maximal).

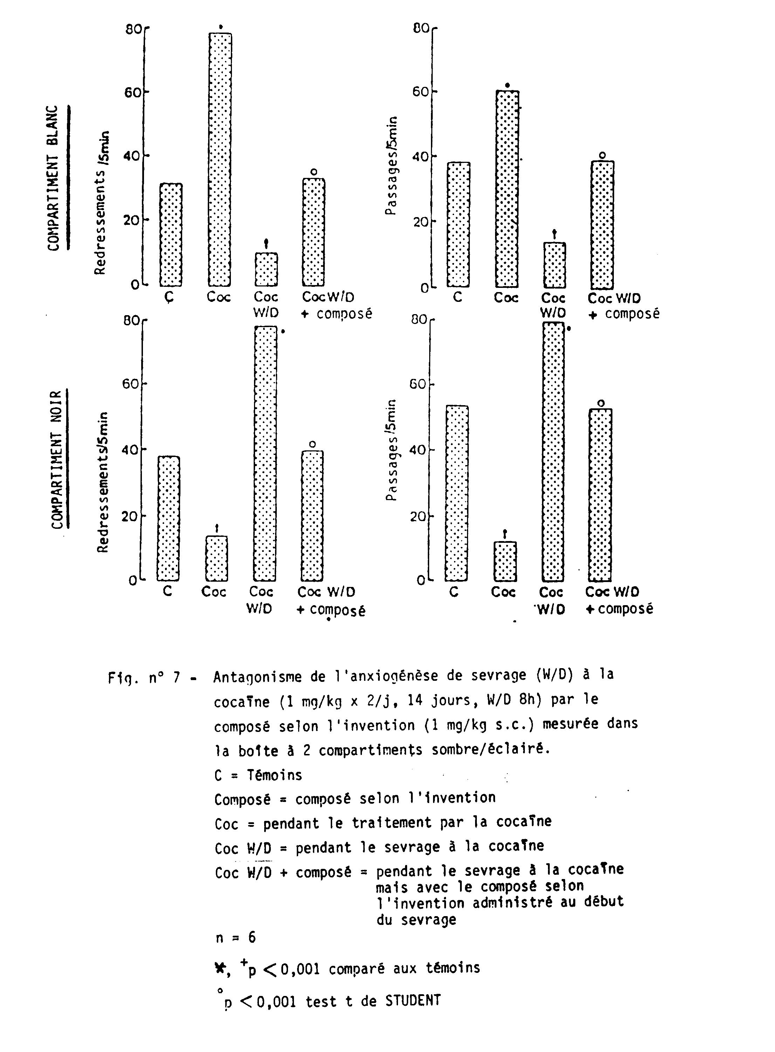

L'administration, au début du sevrage, de 1 mg/kg du composé selon l'invention augmente de façon significative le nombre de passages et de redressements dans le compartiment éclairé par rapport aux animaux sevrés non traités (Fig. n° 7).

### 5. Recherche d'une anxiogénèse de sevrage au composé selon l'invention comparée à celle induite par les benzodiazépines

Certaines substances telles les benzodiazépines induisent une dépendance après utilisation chronique. C'est un de leurs inconvénients en thérapeutique.

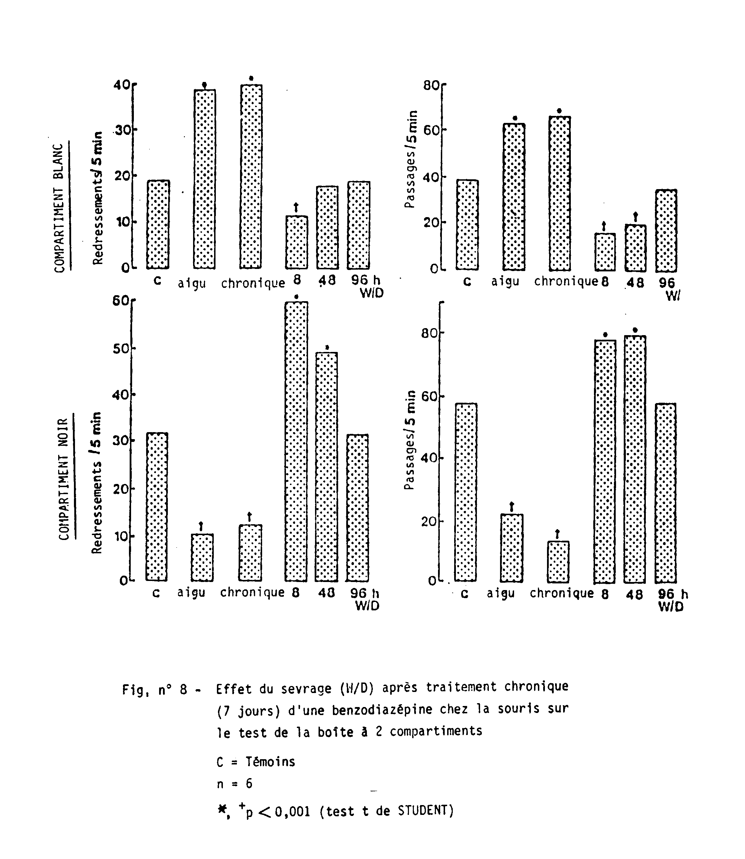

L'administration chronique d'une benzoidazépine pendant 7 jours entraîne, à l'arrêt du traitement une recrudescence de l'anxiété objectivée par un nombre accru de redressements et de passages dans le compartiment sombre (Fig. n° 8).

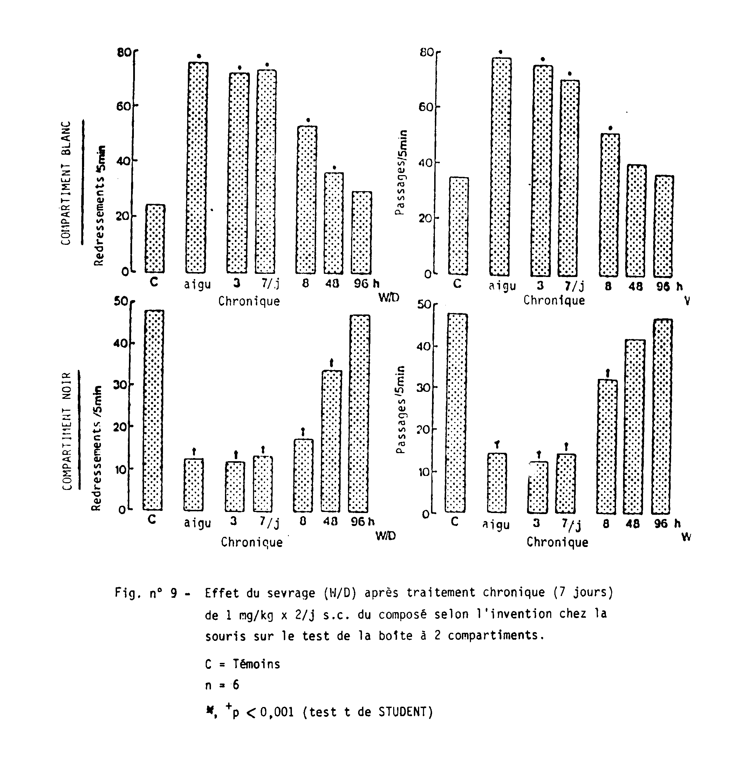

L'administration chronique du composé selon l'invention à la dose de 1 mg/kg x 2/j pendant 7 jours n'induit pas d'anxiété dans les 8, 48 ou 96 heures après l'arrêt du traitement. Au contraire, on constate que l'effet anxiolytique du composé selon l'invention est maintenu après le sevrage, les redressements et passages dans le compartiment éclairé restant significativement plus élevés que ceux des témoins, 8 et 48 heures après le sevrage pour revenir aux valeurs témoins à 96 heures (Fig. n° 9).

### 6. Hyperactivité induite par perfusion intra-accumbens de dopamine chez le rat

Un des facteurs évoqués pour expliquer la génèse de la schizophrénie, serait une hyperactivité dopaminergique cérébrale. La suppression de cette hyperactivité, comme par les neuroleptiques, permettrait une amélioration de l'état psychotique.

Dans le test de l'hyperactivité chez le rat, la dopamine est perfusée par voie intra-cérébrale dans le noyau accumbens à l'aide de mini-pompes osmotiques à une dose de 24 µg/24 heures pendant 13 jours. L'activité motrice spontanée des rats placés dans des cages individuelles est mesurée par l'intermédiaire de cellules photo-électriques. Les résultats sont exprimés en nombre d'interruptions des faisceaux lumineux correspondant aux cellules photo-électriques (passage de l'animal).

Lors de l'administration de la dopamine, l'on observe une augmentation de l'activité locomotrice des animaux qui se manifeste par deux pics d'hyperactivité vers le 3ème et 9ème jour. Les neuroleptiques classiques empêchent la survenue de cette hyperactivité lorsqu'ils sont administrés pendant la période de la perfusion.

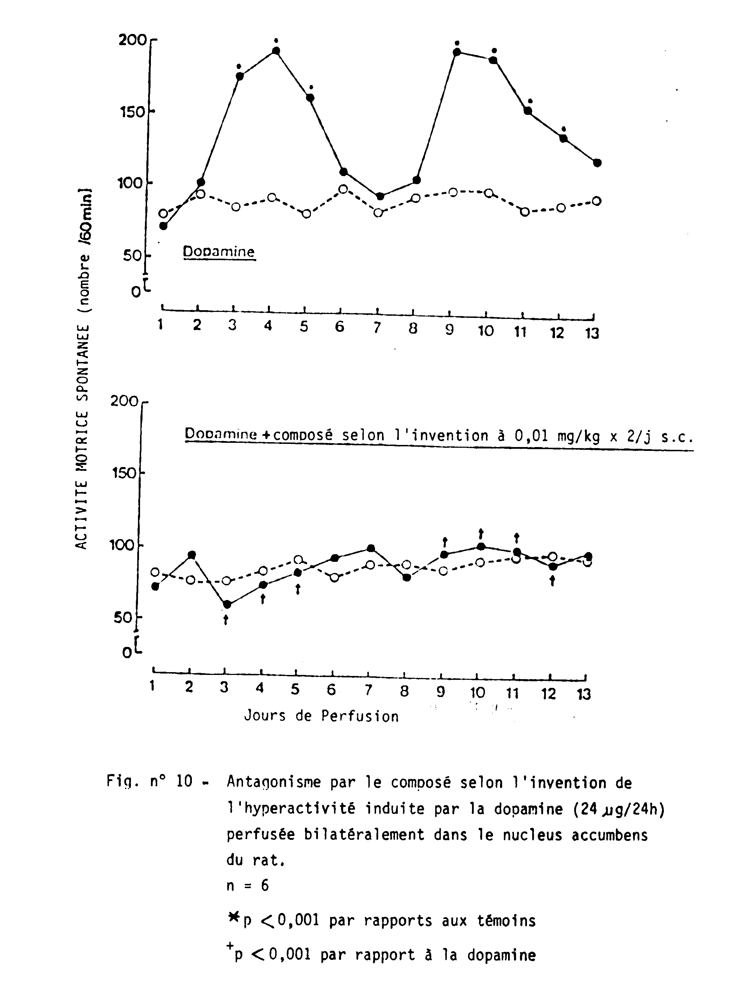

L'administration du composé selon l'invention à une dose de 0,01 mg/kg s.c. x 2/jour prévient l'apparition de l'hyperactivité normalement induite par la perfusion de la dopamine (Fig. n° 10).

Les tests cités ci-dessus démontrent l'activité anxiolytique et antipsychotique du composé selon l'invention. Ces propriétés permettent son utilisation comme médicament dans le traitement des divers états d'anxiété ainsi que dans certains états psychotiques.

Selon les tests, le composé de l'invention se révèle également utile pour faciliter le sevrage de diverses substances et médicaments susceptibles de créer une pharmacodépendance en prévenant l'anxiété engendrée par l'arrêt de ceux-ci.

## Revendications

1. Utilisation du N-[2-(diéthylamino) éthyl] 2-méthoxy 4-[(1-H 4,5-dihydro 2-imidazolyl) amino] 5-chloro benzamide pour la préparation d'un médicament utile comme anxiolytique.

2. Utilisation du N-[2-(diéthylamino) éthyl] 2-méthoxy 4-[(1-H 4,5-dihydro 2-imidazolyl) amino] 5-chloro benzamide pour la préparation d'un médicament utile comme antipsychotique.

## Claims

1. Use of N-[2-(diethylamino)-ethyl]-2-methoxy 4 [(1-H 4,5-dihydro-2-imidazolyl)-amino]-5-chlorobenzamide for the preparation of a medicament useful as an anxiolytic.

2. Use of N-[2-(diethylamino)-ethyl]-2-methoxy 4[(1-H 4,5-dihydro-2-imidazolyl)-amino]-5-chlorobenzamide for the preparation of a medicament useful as an antipsychotic.

## Patentansprüche

1. Verwendung von N-[2-(Diethylamino)-ethyl]-2-methoxy-4-[(1H-4,5-dihydro-2-imidazolyl)-amino]-5-chlorbenzamid für die Herstellung eines als Anxiolytikum verwendbaren Medikaments.

2. Verwendung von N-(2-(Diethylamino)-ethyl]-2-methoxy-4-[(1H-4,5-dihydro-2-imidazolyl)-amino]-5-chlorbenzamid für die Herstellung eines als Antipsychotikum verwendbaren Medikaments.
